# EUROPEAN PATENT APPLICATION

(11) **EP 4 174 168 A1**
(43) Date of publication of application: **03.05.2023**
(21) Application number: 21928345.4
(22) Date of filing: 16.12.2021
(51) Int. Cl.: C12N 1/20, C12N 13/00, A23K 10/18, A23L 29/00, C12R 1/07

(54) **BACILLUS VELEZENSIS BRED BY ULTRASOUND-ASSISTED ADAPTIVE EVOLUTION, AND APPLICATION THEREOF**

(30) Priority: 09.08.2021 CN 202110908586
(71) Applicant: Jiangsu University, Zhenjiang, Jiangsu 212013 (CN)
(72) Inventor: RUAN, Siyu, Zhenjiang, Jiangsu 212013 (CN); MA, Haile, Zhenjiang, Jiangsu 212013 (CN); LI, Yunliang, Zhenjiang, Jiangsu 212013 (CN); JI, Yaoyong, Zhenjiang, Jiangsu 212013 (CN); WANG, Xiaojing, Zhenjiang, Jiangsu 212013 (CN); ZHOU, Anqi, Zhenjiang, Jiangsu 212013 (CN); XIE, Pengfei, Zhenjiang, Jiangsu 212013 (CN); LIU, Xiaoshuang, Zhenjiang, Jiangsu 212013 (CN)
(74) Representative: Frick, Robert
(86) International application number: PCT/CN2021/138824
(87) International publication number: WO 2023/015805

(57) **Abstract**

The present disclosure provides a *Bacillus velezensis* (*B. velezensis*) strain bred through ultrasound-assisted adaptive evolution and use thereof, and belongs to the technical field of microbial engineering. In the present disclosure, a *B. velezensis* strain (*B. velezensis* CICC 23571) is used as a starting strain, and an ultrasound-assisted adaptive evolution process is performed, where a proportion of organic nitrogen in a medium is continuously reduced and proportions of inorganic nitrogen and a sugar source are increased to obtain a *B. velezensis* mutant strain with a high polypeptide yield. Different metabolic pathways in the mutant strain are enhanced, such that the mutant strain can efficiently convert inorganic nitrogen into organic nitrogen or reduce the deaminization of organic nitrogen, and finally a yield of polypeptides is greatly increased.

## Description

### TECHNICAL FIELD

The present disclosure belongs to the technical field of microbial engineering, and in particular relates to a *Bacillus velezensis* (*B. velezensis*) strain bred through ultrasound-assisted adaptive evolution, and use thereof.

### BACKGROUND

A polypeptide is a compound formed by linking amino acids through peptide bonds, which has a size between a size of an amino acid and a size of a protein. In recent years, bioactive polypeptides have been widely used in medicine and health fields such as food, health products, and drugs due to their characteristics such as safety, functionality, and easy absorption.

Currently, main preparation methods of polypeptides include synthesis methods and hydrolysis methods. A microbial fermentation method among the hydrolysis methods is to directly use a protease system produced during a microbial fermentation process to degrade a protein, such that the steps of enzyme production, enzymolysis, and debitterization are performed synchronously, which can not only greatly simplify the production process and reduce the production cost, but also effectively degrade anti-nutritional factors such as glucosinolates and protease inhibitors. However, most of the strains used for the microbial fermentation method are high protease-producing *Bacillus sp.* strains, and such strains have a strong ability to utilize proteins. During a fermentation process, the developed peptide transporters on the cell membrane will transport polypeptides in the product into cells and the polypeptides are used as a nitrogen source to synthesize a bacterial protein, or the polypeptides are deaminized to be used as a carbon source for the growth and reproduction of cells, such that an overall polypeptide yield in a fermentation product is much lower than a theoretical value, which is a bottleneck problem in the industry and is also one of the main issues for restricting the development of polypeptide production through fermentation. Therefore, the breeding of high protease-producing strains with low polypeptide consumption has important scientific values for improving the polypeptide production performance of fermentation thereof.

In recent years, the adaptive laboratory evolution (ALE) technology has become a powerful tool for mutation breeding of industrial microorganisms. In the ALE technology, the quantitative enrichment of a positive mutant is achieved through continuous subculture, and on this basis, a new positive gene mutation is generated to achieve qualitative enrichment, such as to change some phenotypic or physiological characteristics of a strain (such as a bacterial growth rate and a substrate consumption rate). The intrinsic gene mutation and extrinsic environmental screening are the essence of ALE. A rate of evolution depends on a gene mutation rate and screening conditions, but a natural mutation rate of a gene is generally 10⁻⁶ to 10⁻¹⁰, which results in the disadvantages of the relatively low mutation efficiency and the relatively high time cost. Therefore, it is necessary to combine the ALE with a cell sorting technology, a genome modeling technology, and the like to quickly acquire a beneficial mutant phenotype. However, these technologies have high requirements on the experimental equipment and operating level, which is not conducive to industrial promotion and application.

### SUMMARY

In view of the deficiencies in the prior art, the present disclosure provides a *B. velezensis* strain bred through ultrasound-assisted adaptive evolution, and use thereof. In the present disclosure, a *B. velezensis* strain (*B. velezensis* CICC 23571) is used as a starting strain, and an ultrasound-assisted adaptive evolution process is performed, where a proportion of organic nitrogen in a medium is continuously reduced and proportions of inorganic nitrogen and a sugar source are increased to obtain a *B. velezensis* mutant strain with a high polypeptide yield. Different metabolic pathways in the mutant strain are enhanced, such that the mutant strain can efficiently convert inorganic nitrogen into organic nitrogen or reduce the deaminization of organic nitrogen, and finally a yield of polypeptides is greatly increased.

The present disclosure first provides a *B. velezensis* strain with a high polypeptide yield that is bred through ultrasound-assisted adaptive evolution. Specifically, the *B. velezensis* strain includes a *B. velezensis* strain US-ALE-BV3 and a *B. velezensis* strain US-ALE-BV22;
the *B. velezensis* strain US-ALE-BV3 was deposited on July 8, 2021 in the China General Microbiological Culture Collection Center (CGMCC), Beijing, China, with an accession number of CGMCC No. 22846; and
the *B. velezensis* strain US-ALE-BV22 was deposited on July 8, 2021 in the China General Microbiological Culture Collection Center (CGMCC), Beijing, China, with an accession number of CGMCC No. 22847.

The present disclosure also provides a use of the *B. velezensis* strain bred through the ultrasound-assisted adaptive evolution in a food and/or a feed.

Further, the use includes a fermentation with the *B. velezensis* strain bred through the ultrasound-assisted adaptive evolution to produce a polypeptide.

The present disclosure also provides a method for breeding the *B. velezensis* strain described above through ultrasound-assisted adaptive evolution, specifically including the following steps:
subjecting a *B. velezensis* strain at a mid-logarithmic growth phase to a low-intensity ultrasonic treatment at 10 W/mL to 60 W/mL and then to a cold-shock treatment for 0.5 min to 5 min after the low-intensity ultrasonic treatment, and further cultivating the *B. velezensis* strain to implement the breeding through the ultrasound-assisted adaptive evolution.

Further, the low-intensity ultrasonic treatment and the cold-shock treatment after the low-intensity ultrasonic treatment are performed under a condition 1 and a condition 2:
the condition 1: the *B. velezensis* strain is subjected to an ultrasonic treatment at 20 W/mL and 40 kHz for 5 min, then immediately to the cold-shock treatment in an ice-water mixture for 1.5 min, and then to a static cultivation for 40 min at 37°C; and
the condition 2: the *B. velezensis* strain is subjected to an ultrasonic treatment at 40 W/mL and 35 kHz for 10 min, then immediately to the cold-shock treatment in an ice-water mixture for 2.5 min, and then to a static cultivation for 40 min at 37°C; and
during the breeding through the ultrasound-assisted adaptive evolution, the condition 1 and the condition 2 are performed alternately for a total of 20 times.

The present disclosure also provides a microbial preparation including the *B. velezensis* strain. Specifically, the *B. velezensis* strain is the *B. velezensis* strain US-ALE-BV3 or the *B. velezensis* strain US-ALE-BV22.

Further, the microbial preparation is a solid preparation or a liquid preparation.

The present disclosure also provides a use of the microbial preparation described above in a food and/or a feed.

Further, the use includes a fermentation with the microbial preparation to produce a polypeptide.

The present disclosure also provides a method for producing a polypeptide through fermentation, including: inoculating a cell liquid culture of the *B. velezensis* strain bred through the ultrasound-assisted adaptive evolution into a fermentation medium at an inoculum size of 2% to 4% (v/v), and performing fermentation and cultivation in a shaker at 30°C to 35°C and 150 r/min for 24 h.

Further, the fermentation medium includes the following components: a soybean meal or corn gluten meal: 40 g/L to 60 g/L; NaCl: 0.1 g/L to 1.0 g/L; K₂HPO₄: 0.1 g/L to 1.0 g/L; MgSO₄: 0.1 g/L to 1.0 g/L; and FeSO₄: 0.01 g/L to 0.05 g/L.

**Compared with the prior art, the present disclosure has the following beneficial effects.**

In the present disclosure, ultrasonic waves with strong mechanical and biological effects are used to assist the adaptive evolution to breed the *B. velezensis* strain. The method has the following advantages: (1) prominent directionality, strong penetrability, and no contamination to a fermentation broth; (2) continuous mutagenesis, no mutagen and radioactive residue, and high safety; (3) easy equipment amplification and reconstruction, which is suitable for continuous industrial operations; and (4) rapid generation and termination and easy control of ultrasound. In addition, ultrasonic mutagenesis is a weak mutagenesis, and can be used as an external physical field in the ALE technology to construct an efficient and easy-to-operate enrichment screening system, which has important guiding significance for acquiring a high-yield, low-consumption, and high-quality strain.

In the present disclosure, when used for fermentation of a protein and polysaccharide-containing medium, the *B. velezensis* strain bred through ultrasound-assisted adaptive evolution can greatly improve a polypeptide yield, and compared with the original strain, a polypeptide yield of soybean meal fermentation is increased by 3 times or more, and a polypeptide yield of corn gluten meal fermentation is increased by 5 times or more.

The metabolic pathways of the two mutant strains provided by the present disclosure to produce polypeptides using a medium focus on different points, and thus, the two mutant strains can be used for different types of media. In addition, the mutant strains can grow rapidly, which greatly shortens a fermentation period and reduces a time cost, and thus the mutant strains can be used for industrial fermentation production, and can serve as production strains for further research and development.

### BRIEF DESCRIPTION OF THE DRAWINGS

FIG. 1 shows the colony morphology of the *B. velezensis* strain US-ALE-BV3 on a nutrient agar.
FIG. 2 shows the growth curves of the *B. velezensis* strain US-ALE-BV3 and the original strain BV0 under normal growth conditions.
FIG. 3 shows the comparison of polypeptide yield of soybean meal fermentation by the *B*. *velezensis* strain US-ALE-BV3 of the present disclosure and the original strain BV0.
FIG. 4 shows the comparison of polypeptide yield of corn gluten meal fermentation by the *B*. *velezensis* strain US-ALE-BV3 of the present disclosure and the original strain BV0.
FIG. 5 shows the colony morphology of the *B. velezensis* strain US-ALE-BV22 of the present disclosure on a nutrient agar.
FIG. 6 shows the growth curves of the *B. velezensis* strain US-ALE-BV22 of the present disclosure and the original strain BV0 under normal growth conditions.
FIG. 7 shows the comparison of polypeptide yield of soybean meal fermentation by the *B*. *velezensis* strain US-ALE-BV22 of the present disclosure and the original strain BV0.
FIG. 8 shows the comparison of polypeptide yield of corn gluten meal fermentation by the *B*. *velezensis* strain US-ALE-BV22 of the present disclosure and the original strain BV0.

### DETAILED DESCRIPTION OF THE EMBODIMENTS

The present disclosure will be further described below in conjunction with the accompanying drawings and specific examples, but the protection scope of the present disclosure is not limited thereto.

The breeding through the ultrasound-assisted adaptive evolution in the present disclosure has no special limitations on the ultrasound equipment. In the following examples, various processes and methods that are not described in detail are conventional methods well known in the art. The sources, trade names, and components to be listed (if necessary) of the reagents used are indicated when the reagents appear for the first time, and unless otherwise specified, the same reagents appearing thereafter are the same as those indicated for the first time.

The formulas of the media involved in the examples of the present disclosure are as follows:
Luria Bertani (LB) plate: tryptone (Oxoid, UK): 10 g/L, yeast extract (Oxoid, UK): 5 g/L, sodium chloride: 10 g/L, and agar powder: 20 g/L;
LB liquid medium: tryptone (Oxoid, UK): 10 g/L, yeast extract (Oxoid, UK): 5 g/L, and sodium chloride: 10 g/L;
casein medium: Na₂HPO₄•12H₂O: 1.43 g/L, casein: 5 g/L, and KH₂PO₄: 0.36 g/L; and
starch medium: soluble starch: 20 g/L, KNO₃: 1.0 g/L, NaCl: 0.5 g/L, K₂HPO₄: 0.5 g/L, MgSO₄: 0.5 g/L, and FeSO₄: 0.01 g/L.

A CM50 medium, a CM20 medium, and a CM0.5 medium refer to mixed media obtained by mixing the casein medium with the starch medium in volume ratios of 5:5, 2:8, and 0.5:9.5, respectively.

### Example 1 Breeding of a B. velezensis strain through ultrasound-assisted adaptive evolution

A strain *B. velezensis* strain CICC 23571 (purchased from the China Center of Industrial Culture Collection (CICC)) was used as a starting strain (which was recorded as an original strain BV0), and three parallel experiments were performed. A seed culture cultivated for 24 h was inoculated into a CM50 medium at an inoculum size of 2% (v/v), cultivated in a shaker at 33°C and 150 r/min to a mid-logarithmic growth phase (6 h), and then subjected to a low-intensity ultrasonic treatment (10 W/mL to 60 W/mL) and a short-time cold-shock treatment (0.5 min to 5 min) to achieve the breeding through the ultrasound-assisted adaptive evolution, and experimental parameters were optimized to obtain the optimal conditions for the ultrasonic treatment and the cold-shock treatment.

The optimal conditions for the ultrasonic treatment and the cold-shock treatment included a condition 1 and a condition 2:
the condition 1: the *B. velezensis* strain was subjected to an ultrasonic treatment at 20 W/mL and 40 kHz for 5 min, then immediately to the cold-shock treatment in an ice-water mixture for 1.5 min, and then to a static cultivation for 40 min at 37°C; and
the condition 2: the *B. velezensis* strain was subjected to an ultrasonic treatment at 40 W/mL and 35 kHz for 10 min, then immediately to the cold-shock treatment in an ice-water mixture for 2.5 min, and then to a static cultivation for 40 min at 37°C.

During the breeding through the ultrasound-assisted adaptive evolution, the ultrasonic treatment and the cold-shock treatment were performed under the condition 1 on day 1, and were performed under the condition 2 on day 2, and were performed under the condition 1 on day 3, and were performed under the condition 2 on day 4, and then the two conditions were performed alternately every other day, that is, the ultrasonic treatment and the cold-shock treatment were performed under the condition 1 on odd days and were performed under the condition 2 on even days. The ultrasonic treatment and the cold-shock treatment were performed for a total of 40 days, including three stages.

A first stage: On day 1, a CM50 culture was subjected to the ultrasonic treatment and the cold-shock treatment under the condition 1, and then cultivated in a shaker at 33°C and 150 r/min for 24 h; and on day 2, a bacterial suspension of day 1 was inoculated (inoculum size: 2%, v/v) into a fresh CM50 liquid medium, cultivated to a mid-logarithmic growth phase (6 h), subjected to the ultrasonic treatment and the cold-shock treatment under the condition 2, and then further cultivated in a shaker at 33°C and 150 r/min for 24 h. That is, a culture of the previous day was used as a seed culture of the next day, and the above operations were performed repeatedly for a total of 8 days (CM50 medium adaptation stage).

A second stage: On day 9, a seed culture of day 8 (inoculum size: 2%, v/v) was inoculated into a fresh CM20 liquid medium and cultivated, during which the fresh CM20 medium was changed and the ultrasonic treatment and the cold-shock treatment were performed every day. Similar to the first stage, the conditions 1 and 2 were performed alternately for a total of 12 days (CM20 medium adaptation stage).

A third stage: On day 21, a seed culture of day 20 (inoculum size: 2%, v/v) was inoculated into a fresh CM0.5 liquid medium and cultivated, during which the fresh CM0.5 medium was changed and the ultrasonic treatment and the cold-shock treatment were performed every day. Similar to the first stage, the conditions 1 and 2 were performed alternately for a total of 20 days (CM0.5 medium adaptation stage) until the 40-day evolutionary mutagenesis was completed.

Mutant strains finally obtained by screening were streaked on a casein solid plate and statically cultivated at 33°C for 24 h, and then according to growth conditions, single-colony mutant strains with strong protease-producing ability (larger transparent circle) and robust growth situation were selected and marked as *B. velezensis* strain US-ALE-BV3 and *B. velezensis* strain US-ALE-BV22, respectively.

### Example 2 Morphological observation and 16s rDNA identification of the B. velezensis strain US-ALE-BV3

### (1) Morphological observation of the B. velezensis strain US-ALE-BV3

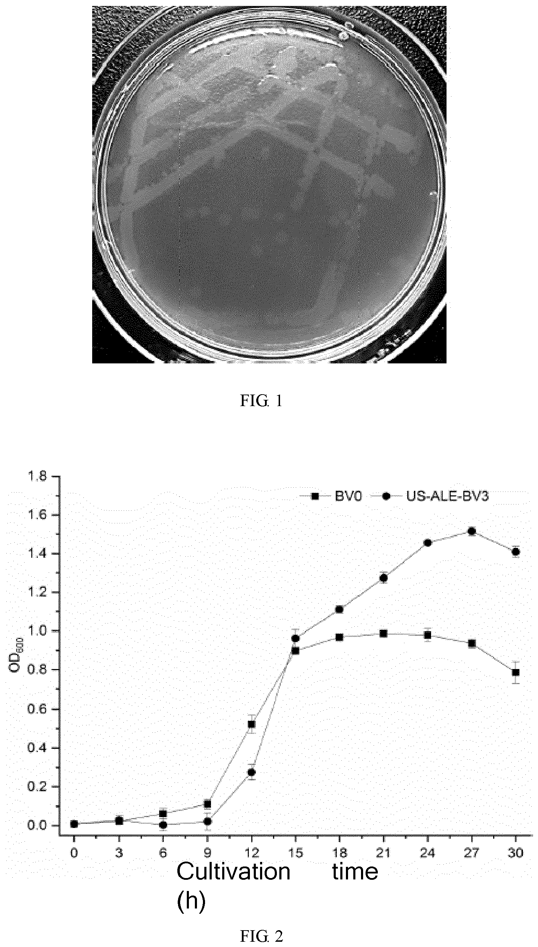

In this example, the *B. velezensis* strain US-ALE-BV3 was subjected to morphological observation. FIG. 1 shows the colony morphology of the *B. velezensis* strain US-ALE-BV3 on a nutrient agar, and it can be seen from the figure that the US-ALE-BV3 colony is milky-white and translucent and has a small number of bulges and folds, and compared with the original strain, the US-ALE-BV3 colony is thicker, and has a smaller colony diameter and a less obvious jagged edge.

### (2) 16s rDNA identification of the B. velezensis strain US-ALE-BV3

In this example, the *B. velezensis* strain US-ALE-BV3 was also subjected to 16s rDNA strain identification, and specific steps were as follows: the whole genome of the *B. velezensis* strain US-ALE-BV3 was extracted with a genome kit (TaKaRa PrimeSTAR Max Premix), and then the universal primers 27F: 5'-AGAGTTTGATCCTGGCTCAG-3' (SEQ ID No: 1) and 1492R: 5'-TACGGYTACCTTGTTACGACTT-3' (SEQ ID No: 2) were used to acquire a 16s rDNA fragment of the strain, and a sequencing result thereof was subjected to alignment on the National Center for Biotechnology Information (NCBI), and it was found that a matching degree with the *B. velezensis* strain SCGB 574 reached 99.87%, indicating that the mutant strain was a *B. velezensis* mutant strain, which was named US-ALE-BV3 and had a sequence set forth in SEQ ID No: 3.

The *B. velezensis* strain US-ALE-BV3 was deposited on July 8, 2021 in the China General Microbiological Culture Collection Center (CGMCC), Beijing, China, with an accession number of CGMCC No. 22846.

### Example 3 Comparison of growth of the B. velezensis strain US-ALE-BV3 and the original strain BV0

In this example, the growth of the *B. velezensis* strain US-ALE-BV3 and the growth of the original strain BV0 (*B. velezensis* CICC 23571) were investigated and compared, and specific steps were as follows:
(1) The original *B. velezensis* strain BV0 and the *B. velezensis* strain US-ALE-BV3 obtained in Example 1 were each inoculated into an LB liquid medium for activation, and cultivated overnight in a shaker at 33°C and 150 r/m.
(2) A seed culture obtained in step (1) was inoculated into 50 mL of a casein/starch mixed medium (3:1, v/v) (250 mL Erlenmeyer flask) at an inoculum size of 1% (v/v), and cultivated in a shaker at 33°C and 150 r/min.
(3) During the cultivation process in step (2), a sample was collected every 3 h, and an OD value at 600 nm was determined. Growth curves were plotted.

FIG. 2 shows the growth curves of the *B. velezensis* strain US-ALE-BV3 and the original strain BV0 under normal growth conditions, and it can be seen from the growth performance test analysis in the figure that a growth curve of the *B. velezensis* strain US-ALE-BV3 is significantly different from a growth curve of the original strain BV0, and compared with the original strain BV0, the *B. velezensis* strain US-ALE-BV3 has a longer logarithmic growth phase, a shorter stationary phase, and a significantly higher growth rate in the fermentation anaphase.

### Example 4 Production of polypeptides through fermentation of soybean meal with the B. velezensis strain US-ALE-BV3 and the original strain BV0

In this example, the *B. velezensis* strain US-ALE-BV3 and the original strain BV0 were each used for fermentation of soybean meal to produce polypeptides, and specific steps were as follows:

### (1) Preparation of cell liquid cultures of the B. velezensis strain US-ALE-BV3 and the original strain BV0:

The original *B. velezensis* strain BV0 and the *B. velezensis* strain US-ALE-BV3 obtained in Example 1 were each inoculated into an LB liquid medium for activation, and cultivated overnight in a shaker at 33°C and 150 r/m to obtain the cell liquid cultures of the *B. velezensis* strain US-ALE-BV3 and the original strain BV0.

### (2) Fermentation test:

The cell liquid cultures of the above strains were each inoculated into a 250 mL Erlenmeyer flask filled with 50 mL of a sterilized fermentation medium at an inoculum size of 2% (v/v), and cultivated in a shaker at 33°C and 150 r/min for 24 h to obtain fermentation broths.

The fermentation medium included the following components: soybean meal: 40 g/L to 60 g/L; NaCl: 0.1 g/L to 1.0 g/L; K₂HPO₄: 0.1 g/L to 1.0 g/L; MgSO₄: 0.1 g/L to 1.0 g/L; and FeSO₄: 0.01 g/L to 0.05 g/L, which were mixed and sterilized at 121°C under high-pressure steam for 20 min.

### (3) Determination of polypeptide contents:

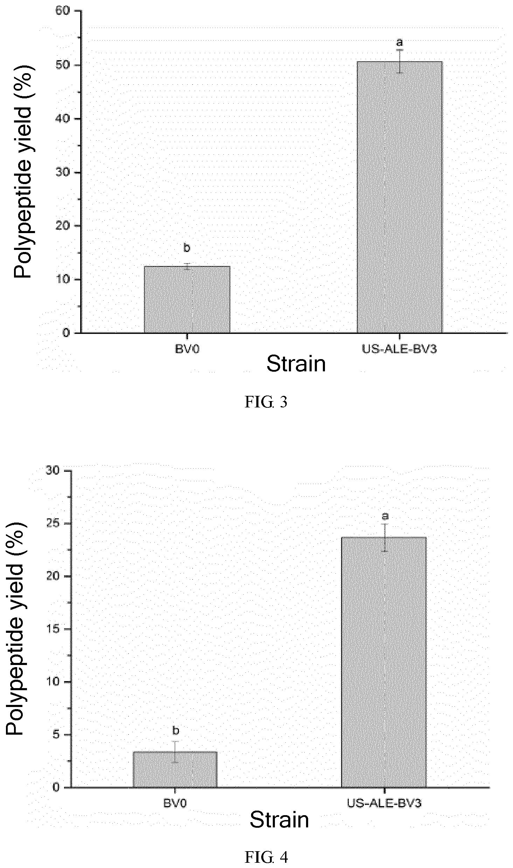

The fermentation broths obtained in step (2) were each centrifuged at 4,000 r/min for 10 min to obtain a fermentation supernatant, a 15% (w/v) trichloroacetic acid (TCA) solution was mixed with the fermentation supernatant in equal volumes, and a resulting mixture was allowed to stand for 10 min and then centrifuged at 4,000 r/min for 10 min; and a resulting precipitate was discarded, and then the Kjeldahl method was used to determine a polypeptide content in the fermentation supernatant. Polypeptide yields of the original strain BV0 and the mutant strain US-ALE-BV3 were calculated, and results were shown in FIG. 3.

It can be seen from FIG. 3 that polypeptide yields of soybean meal fermentation with the BV0 and US-ALE-BV3 are 12.44% and 50.63%, respectively, and a polypeptide yield of soybean meal fermentation with the *B. velezensis* strain US-ALE-BV3 is 4.07 times higher than that of the original strain BV0.

### Example 5 Production of polypeptides through fermentation of corn gluten meal with the B. velezensis strain US-ALE-BV3 and the original strain BV0

### (1) Preparation of cell liquid cultures of the B. velezensis strain US-ALE-BV3 and the original strain BV0:

The original *B. velezensis* strain BV0 and the mutant strain US-ALE-BV3 obtained in Example 1 were each inoculated into an LB liquid medium for activation, and cultivated overnight in a shaker at 33°C and 150 r/m to obtain the cell liquid cultures of the *B. velezensis* strain US-ALE-BV3 and the original strain BV0.

### (2) Fermentation test:

The cell liquid cultures of the above strains were each inoculated into a 250 mL Erlenmeyer flask filled with 50 mL of a sterilized fermentation medium at an inoculum size of 2% (v/v), and cultivated in a shaker at 33°C and 150 r/min for 24 h to obtain fermentation broths.

The fermentation medium included the following components: corn gluten meal: 40 g/L to 60 g/L; NaCl: 0.1 g/L to 1.0 g/L; K₂HPO₄: 0.1 g/L to 1.0 g/L; MgSO₄: 0.1 g/L to 1.0 g/L; and FeSO₄: 0.01 g/L to 0.05 g/L, which were mixed and sterilized at 121°C under high-pressure steam for 20 min.

### (3) Determination of polypeptide contents:

The fermentation broths obtained in step (2) were each centrifuged at 4,000 r/min for 10 min to obtain a fermentation supernatant, a 15% (w/v) TCA solution was mixed with the fermentation supernatant in equal volumes, and a resulting mixture was allowed to stand for 10 min and then centrifuged at 4,000 r/min for 10 min; and a resulting precipitate was discarded, and then the Kjeldahl method was used to determine a polypeptide content in the fermentation supernatant. Polypeptide yields of the original strain BV0 and the mutant strain US-ALE-BV3 were calculated, and results were shown in FIG. 4.

It can be seen from FIG. 4 that polypeptide yields of corn gluten meal fermentation with the BV0 and US-ALE-BV3 are 3.34% and 23.67%, respectively, and a polypeptide yield of corn gluten meal fermentation with the mutant strain US-ALE-BV3 is 7.09 times higher than that of the original strain BV0.

### Example 6 Morphological observation and 16s rDNA identification of the B. velezensis strain US-ALE-BV22

### (1) Morphological observation of the B. velezensis strain US-ALE-BV22

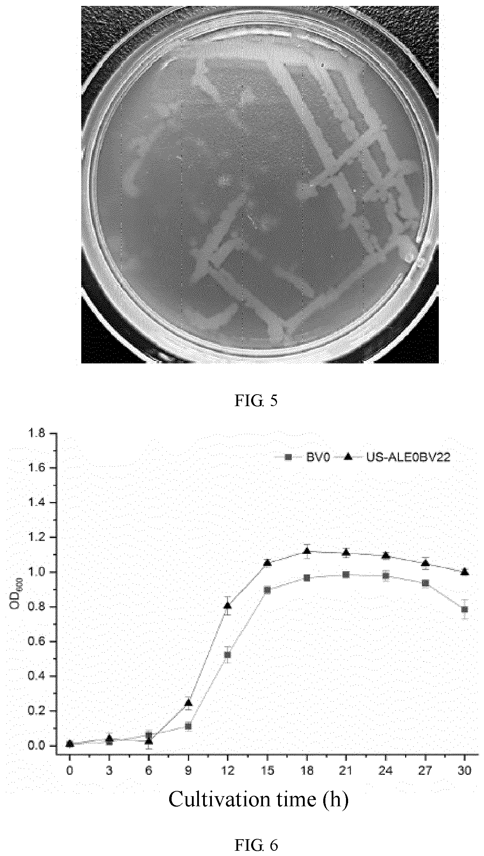

In this example, the *B. velezensis* strain US-ALE-BV22 was subjected to morphological observation. FIG. 5 shows the colony morphology of the *B. velezensis* strain US-ALE-BV22 on a nutrient agar, and it can be seen from the figure that the US-ALE-BV22 colony is milky-white and translucent and has a small number of bulges and folds; and compared with the original strain, the US-ALE-BV22 colony is thicker, and has a smaller colony diameter and a less obvious jagged edge.

### (2) 16s rDNA identification of the B. velezensis strain US-ALE-BV22

In this example, the *B. velezensis* strain US-ALE-BV22 was also subjected to 16s rDNA strain identification, and specific steps were as follows: the whole genome of the *B. velezensis* strain US-ALE-BV22 was extracted with a genome kit (TaKaRa PrimeSTAR Max Premix), and then the universal primers 27F: 5'-AGAGTTTGATCCTGGCTCAG-3' (SEQ ID No: 1) and 1492R: 5'-TACGGYTACCTTGTTACGACTT-3' (SEQ ID No: 2) were used to acquire a 16s rDNA fragment of the strain; and a sequencing result thereof was subjected to alignment on NCBI, and it was found that a matching degree with the *B. velezensis* strain SCGB 574 reached 99.87%, indicating that the mutant strain was a *B. velezensis* mutant strain, which was named US-ALE-BV22 and had a sequence set forth in SEQ ID No: 4.

The *B. velezensis* strain US-ALE-BV22 was deposited on July 8, 2021 in the China General Microbiological Culture Collection Center (CGMCC), Beijing, China, with an accession number of CGMCC No. 22847.

### Example 7 Growth of the B. velezensis strain US-ALE-BV22 and the original strain BV0

In this example, the growth of the *B. velezensis* strain US-ALE-BV22 and the growth of the original strain BV0 were investigated and compared, and specific steps were as follows:
(1) The original *B. velezensis* strain BV0 and the mutant strain US-ALE-BV22 obtained in Example 1 were each inoculated into an LB liquid medium for activation, and cultivated overnight in a shaker at 33°C and 150 r/m.
(2) A seed culture obtained in step (1) was inoculated into 50 mL of a casein/starch mixed medium (3:1) (250 mL Erlenmeyer flask) at an inoculum size of 1% (v/v), and cultivated in a shaker at 33°C and 150 r/min.
(3) During the cultivation process in step (2), a sample was collected every 3 h, and an OD value at 600 nm was determined. Growth curves were plotted.

FIG. 6 shows the growth curves of the *B. velezensis* strain US-ALE-BV22 and the original strain BV0 under normal growth conditions. It can be seen from the figure that a trend of a growth curve of the mutant strain US-ALE-BV22 is basically the same as that of the original strain BV0, but a growth rate of the mutant strain is generally higher than that of BV0.

### Example 8 Production of polypeptides through fermentation of soybean meal with the B. velezensis strain US-ALE-BV22 and the original strain BV0

In this example, the *B. velezensis* strain US-ALE-BV22 and the original strain BV0 were each used for fermentation of soybean meal to produce polypeptides, and specific steps were as follows:

### (1) Preparation of cell liquid cultures of the B. velezensis strain US-ALE-BV22 and the original strain BV0:

The original *B. velezensis* strain BV0 and the *B. velezensis* strain US-ALE-BV22 obtained in Example 1 were each inoculated into an LB liquid medium for activation, and cultivated overnight in a shaker at 33°C and 150 r/m to obtain the cell liquid cultures of the *B. velezensis* strain US-ALE-BV22 and the original strain BV0.

### (2) Fermentation test:

The cell liquid cultures of the above strains were each inoculated into a 250 mL Erlenmeyer flask filled with 50 mL of a sterilized fermentation medium at an inoculum size of 2% (v/v), and cultivated in a shaker at 33°C and 150 r/min for 24 h to obtain fermentation broths.

The fermentation medium included the following components: soybean meal: 40 g/L to 60 g/L; NaCl: 0.1 g/L to 1.0 g/L; K₂HPO₄: 0.1 g/L to 1.0 g/L; MgSO₄: 0.1 g/L to 1.0 g/L; and FeSO₄: 0.01 g/L to 0.05 g/L, which were mixed and sterilized at 121°C under high-pressure steam for 20 min.

### (3) Determination of polypeptide contents:

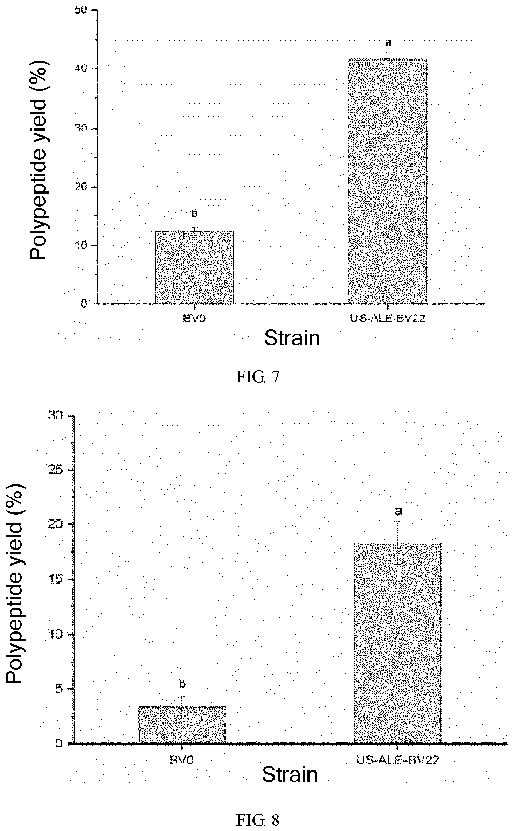

The fermentation broths obtained in step (2) were each centrifuged at 4,000 r/min for 10 min to obtain a fermentation supernatant, a 15% (w/v) TCA solution was mixed with the fermentation supernatant in equal volumes, and a resulting mixture was allowed to stand for 10 min and then centrifuged at 4,000 r/min for 10 min; and a resulting precipitate was discarded, and then the Kjeldahl method was used to determine a polypeptide content in the fermentation supernatant. Polypeptide yields of the original strain BV0 and the mutant strain US-ALE-BV22 were calculated, and results were shown in FIG. 7.

It can be seen from FIG. 7 that polypeptide yields of soybean meal fermentation with the BV0 and US-ALE-BV22 are 12.44% and 41.67%, respectively; and a polypeptide yield of soybean meal fermentation with the mutant strain US-ALE-BV22 is 3.35 times higher than that of the original strain BV0.

### Example 9 Production of polypeptides through fermentation of corn gluten meal with the B. velezensis strain US-ALE-BV22 and the original strain BV0

### (1) Preparation of cell liquid cultures of the B. velezensis strain US-ALE-BV22 and the original strain BV0:

The original *B. velezensis* strain BV0 and the mutant strain US-ALE-BV22 obtained in Example 1 were each inoculated into an LB liquid medium for activation, and cultivated overnight in a shaker at 33°C and 150 r/m to obtain the cell liquid cultures of the *B. velezensis* strain US-ALE-BV22 and the original strain BV0.

### (2) Fermentation test:

The cell liquid cultures of the above strains were each inoculated into a 250 mL Erlenmeyer flask filled with 50 mL of a sterilized fermentation medium at an inoculum size of 2% (v/v), and cultivated in a shaker at 33°C and 150 r/min for 24 h to obtain fermentation broths.

The fermentation medium included the following components: corn gluten meal: 40 g/L to 60 g/L; NaCl: 0.1 g/L to 1.0 g/L; K₂HPO₄: 0.1 g/L to 1.0 g/L; MgSO₄: 0.1 g/L to 1.0 g/L; and FeSO₄: 0.01 g/L to 0.05 g/L, which were mixed and sterilized at 121°C under high-pressure steam for 20 min.

### (3) Determination of polypeptide contents:

The fermentation broths obtained in step (2) were each centrifuged at 4,000 r/min for 10 min to obtain a fermentation supernatant, a 15% (w/v) TCA solution was mixed with the fermentation supernatant in equal volumes, and a resulting mixture was allowed to stand for 10 min and then centrifuged at 4,000 r/min for 10 min; and a resulting precipitate was discarded, and then the Kjeldahl method was used to determine a polypeptide content in the fermentation supernatant. Polypeptide yields of the original strain BV0 and the mutant strain US-ALE-BV22 were calculated, and results were shown in FIG. 8.

It can be seen from FIG. 8 that polypeptide yields of corn gluten meal fermentation with the BV0 and US-ALE-BV22 are 3.34% and 18.33%, respectively; and a polypeptide yield of corn gluten meal fermentation with the mutant strain US-ALE-BV22 is 5.49 times higher than that of the original strain BV0.

It is verified through experiments that, when the mutant strains US-ALE-BV3 and US-ALE-BV22 screened by the present disclosure are used for fermentation of soybean meal and corn gluten meal, a polypeptide yield can be greatly increased. Compared with the original strain, a polypeptide yield of soybean meal fermentation is increased 3 times or more, and a polypeptide yield of corn gluten meal fermentation is increased 5 times or more. As metabolic pathways of polypeptide production with different media emphasize on different points, the two mutant strains provided by the present disclosure can be used for different types of media. In addition, the mutant strains can grow rapidly, which greatly shortens a fermentation period and reduces a time cost; and thus the mutant strains can be used for industrial fermentation production, and can serve as production strains for further research and development.

The above examples are preferred implementations of the present disclosure, but the present disclosure is not limited to the above implementations. Any obvious improvement, substitution, or modification made by those skilled in the art without departing from the essence of the present disclosure should fall within the protection scope of the present disclosure.

## Claims

1. A *Bacillus velezensis* strain bred through an ultrasound-assisted adaptive evolution, wherein the *Bacillus velezensis* strain has an ability to improve a polypeptide yield.

2. The *Bacillus velezensis* strain according to claim 1, wherein the *Bacillus velezensis* strain comprises a *Bacillus velezensis* strain US-ALE-BV3 and a *Bacillus velezensis* strain US-ALE-BV22;
the *Bacillus velezensis* strain US-ALE-BV3 has an accession number of CGMCC No. 22846; and
the *Bacillus velezensis* strain US-ALE-BV22 has an accession number of CGMCC No. 22847.

3. A use of the *Bacillus velezensis* strain according to claim 1 in a food and/or a feed.

4. The use according to claim 3, wherein the use comprises a production of a polypeptide through a fermentation.

5. A method for breeding the *Bacillus velezensis* strain according to claim 1 through the ultrasound-assisted adaptive evolution, comprising the following steps:
subjecting a *Bacillus velezensis* strain at a mid-logarithmic growth phase to a low-intensity ultrasonic treatment at 10 W/mL to 60 W/mL and then to a cold-shock treatment for 0.5 min to 5 min after the low-intensity ultrasonic treatment, and further cultivating the *Bacillus velezensis* strain to implement the breeding through the ultrasound-assisted adaptive evolution.

6. The method for breeding the *Bacillus velezensis* strain through the ultrasound-assisted adaptive evolution according to claim 4, wherein the low-intensity ultrasonic treatment and the cold-shock treatment are performed under a condition 1 and a condition 2, and during the breeding through the ultrasound-assisted adaptive evolution, the condition 1 and the condition 2 are performed alternately for a total of 20 times:
the condition 1: the *Bacillus velezensis* strain is subjected to an ultrasonic treatment at 20 W/mL and 40 kHz for 5 min, then immediately to the cold-shock treatment in an ice-water mixture for 1.5 min, and then to a static cultivation for 40 min at 37°C; and
the condition 2: the *Bacillus velezensis* strain is subjected to an ultrasonic treatment at 40 W/mL and 35 kHz for 10 min, then immediately to the cold-shock treatment in an ice-water mixture for 2.5 min, and then to a static cultivation for 40 min at 37°C.

7. A microbial preparation, comprising the *Bacillus velezensis* strain according to claim 1.

8. The microbial preparation according to claim 7, wherein the microbial preparation is a solid preparation or a liquid preparation.

9. A use of the microbial preparation according to claim 7 or 8 in a food and/or a feed.

10. The use according to claim 9, wherein the use comprises a production of a polypeptide through a fermentation.
